(19) Europäisches Patentamt
European Patent Office
Office européen des brevets

(11) Publication number: **0 080 047**
**B1**

(12) # EUROPEAN PATENT SPECIFICATION

(45) Date of publication of patent specification: **15.05.85**

(51) Int. Cl.[4]: **A 61 K 31/485** //
(A61K31/485, 31:40)

(21) Application number: **82109055.2**

(22) Date of filing: **30.09.82**

(54) **Analgesic compositions containing an opiate alkaloid compound and a carbazole compound.**

(30) Priority: **23.11.81 US 323834**

(43) Date of publication of application:
**01.06.83 Bulletin 83/22**

(45) Publication of the grant of the patent:
**15.05.85 Bulletin 85/20**

(84) Designated Contracting States:
**CH DE FR IT LI**

(56) References cited:
**US-A-3 896 145**

**CHEMICAL ABSTRACTS, vol. 85, no. 5, 2nd August 1976, page 26, col. 2, no. 28631k, Columbus Ohio (USA); L.O. RANDALL et al.: "Analgesic and anti-inflammatory activity of 6-chloro-alpha-methyl-carbazole-2-acetic acid (C-5720)**

(73) Proprietor: **F. HOFFMANN-LA ROCHE & CO. Aktiengesellschaft**
**CH-4002 Basel (CH)**

(72) Inventor: **Baruth, Herman William, Jr.**
**3 Varick Street**
**Wayne, N.J. (US)**
Inventor: **Berger, Leo**
**7 Parkway**
**Montclair, N.J. (US)**
Inventor: **Corraz, Alfred J.**
**17 Valley View Terrace**
**Wayne, N.J. (US)**
Inventor: **Sepinwall, Jerry**
**5 Stuart Court**
**Pine Brook, N.J. (US)**

(74) Representative: **Bertschinger, Christoph, Dr. et al**
**Grenzacherstrasse 124 P.O. Box 3255**
**CH-4002 Basel (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

The present invention relates to analgesic compositions containing as active ingredients an opiate alkaloid compound of the formula

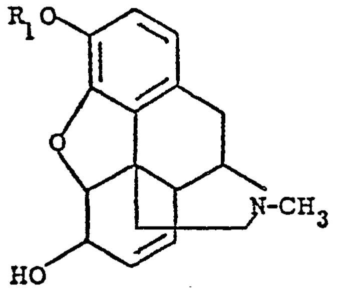 I, or 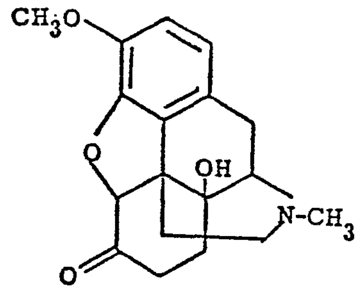 II

wherein $R_1$ is hydrogen or alkyl containing 1—7 carbon atoms, or a pharmaceutically acceptable acid addition salt thereof; and the carbazole compound 6-chloro-α-methyl-carbazole-2-acetic acid of the formula

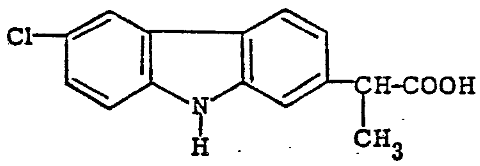 III

or a salt thereof with a pharmaceutically acceptable base.

The alkyl group may be straight or branched chain, for example, methyl, ethyl, propyl, isopropyl, butyl, isobutyl, tertiary butyl, isopentyl, pentyl, or heptyl.

Relief of pain is one of the important goals in medicine. One method for alleviating pain is administration of analgesic drugs which act to decrease the awareness of the sensation of pain by elevating the pain threshold. Opiate alkaloids, for example, morphine, codeine and oxycodone, are among the more potent analgesic drugs available in the treatment of pain. Although the primary action of opiate alkaloids is analgesia, there are serious side-effects associated with these compounds such as respiratory depression and addiction.

The carbazole compound of formula III potentiates the analgesic action of the aforementioned opiate alkaloids and, thereby, reduces the amount of opiate used. Thus, the maximum analgesic affect of these opiate alkaloids is maintained and their side-effects are minimized.

The opiate compounds of formulas I and II are known compounds having analgesic activity. ["Remington's Pharmaceutical Sciences", 13th Edition, E. W. Martin, Editor-in-Chief, Chapter 70, Mack Publishing Co., Easton, Pennsylvania (1965)]. Exemplary of the opiates of formula I and II are codeine, morphine and oxycodone.

The term "pharmaceutically acceptable acid addition salts" utilized in connection with the opiate compounds denotes salts derived from inorganic acids, such as hydrochloric acid, hydrobromic acid, sulfuric acid and phosphoric acid; and organic acids such as acetic acid, citric acid, lactic acid, maleic acid, salicylic acid, succinic acid and p-toluenesulfonic acid.

The carbazole compound, 6-chloro-α-methyl-carbazole-2-acetic acid, of formula III utilized in the compositions of the invention is a known compound having analgesic, anti-inflammatory and anti-rheumatic activity (U.S. Patent 3,896,145). The preferred compound of formula III is the racemic material, however, the d- or l-isomers may also be used in the present invention.

In connection with the carbazole compound, the term "salt thereof with a pharmaceutically acceptable base" denotes salts derived from alkali metal hydroxides, such as sodium hydroxide or potassium hydroxide; alkaline earth metal hydroxides, such as calcium hydroxide or barium hydroxide; sodium alkoxides, such as sodium ethylate or potassium ethylate; organic bases such as substituted ammonium compounds, piperidine, diethanolamine or N-methylglucamine.

The compositions of this invention can be prepared by bringing the active ingredients into a galenical dosage form. According to conventional procedures recognized in the art the active components can be mixed with pharmaceutical, therapeutically inert inorganic or organic carrier materials. Such compositions may be in liquid form, that is as solutions, suspensions or emulsions, or in solid form, for example, tablets, troches or capsules.

Suitable art-recognized therapeutically inert pharmaceutical carrier materials useful in the preparation of the compositions of the present invention include, for example, water, gelatin, gum arabic, lactose, starch, magnesium stearate, talc, vegetable oils and polyalkyleneglycols. The pharmaceutical compositions of this invention may be sterilized and may contain art-recognized adjuvants, for example,

preservatives, stablizers, wetting or emulsifying agents, agents for flavor improvement, salts to adjust osmotic pressure and buffers.

A combination of an opiate compound of formula I or II and the carbazole compound of formula III can be used in a form adapted for oral administration. The ratio of the carbazole compound of formula III to an opiate compound of formula I or II can vary within a wide range, for example in the range of from about 1:10 (parts by weight) to about 10:1 (parts by weight), the preferred ratio being in the range of from about 1:5 to about 5:1. A preferred ratio for the combination of a carbazole compound of formula III and codeine is 5:2, carbazole to codeine. The analgesic compositions of this invention may contain about 0,05 to 90 percent by weight of the mixture of the active ingredients.

Unexpectedly, the analgesic effect observed when an opiate alkaloid of formula I or II is combined with the carbazole compound of formula III is significantly greater than that which would result from the additive effect of the separate components. The advantages of the analgesic potentiation are many, and include a reduction in the dosage or the opiate component required to produce analgesia, with a consequent reduction in undesirable side effects. The effect occurs at a constant magnitude that represents approximately two- or three-fold increase in potency, and it represents a selective effect.

The potentiation effect can be demonstrated utilizing the procedure hereinafter described. Five male mice weighing from 20 to 22 grams were used per dose level. Five vehicle treated mice typically produce 100 to 130 writhing episodes during the five-minute observation period, beginning 10 minutes after the intraperitoneal injection of 0.25 ml of an 0.02% aqueous ethanol solution of 2-phenyl-1,4-benzoquinone, hereinafter referred to as phenylquinone. The phenylquinone was dissolved in 5% ethyl alcohol and distilled water, gently heated to 37°C and kept in a brown stoppered bottle to prevent deterioration. The test compounds were administered orally, in rapid succession, in distilled water 15 minutes prior to the phenylquinone.

The protection (percent of inhibition of writhing) afforded by an analgesic substance was calculated for each experimental group of five mice as follows:

$$\text{Percent Inhibition} = 100 \times \frac{(\#\text{ Writhes by Control Group} - \#\text{ Writhes by Test Group})}{\#\text{ Writhes by Control Group}}$$

Regression Analysis and Fieller's Theorem were used to compute the $ED_{50}$ and its 95% confidence limits. The analysis program made use of the number of writhes exhibited by each mouse in each treatment group, and it estimated the dose associated with 50% of the average number of writhes in the vehicle control group ($ED_{50}$). The means and standard deviations computed during this analysis were then analyzed by "parallel line bioassay" that compared the opiate alkaloid alone in graded doses against, in turn, each combination of a range of opiate alkaloid doses plus a constant dose of carbazole compound. Provided that no significant nonparallelism was found in any comparison, a relative potency estimate and its 95% Fieller's limits were computed. The opiate alkaloid dose scale here was always logarithmic.

The sequence of treatments in this study was not random. Rather, the $ED_{50}$ for the opiate alkaloid alone was determined. After this, combination experiments were conducted in which the carbazole compound rac. 6-chloro-α-methyl-carbazole-2-acetic acid, was administered at a constant dose and the $ED_{50}$ of the opiate alkaloid was redetermined in the presence of racemic 6-chloro-α-methyl-carbazole-2-acetic acid. Initially, a dose level of carbazole (1 mg/kg p.o.) that was approximately one-fiftieth of the $ED_{50}$ for racemic 6-chloro-α-methyl-carbazole-2-acetic acid alone was selected for the combination experiments. The results for codeine are set forth in Table I.

TABLE I

Summary of Analgesic Effects of Codeine and 6-Chloro-α-methylcarbazole-2-acetic acid (Compound A) and Their Combinations in the Phenylquinone Writhing Test

| Compound/Combination | $ED_{50}$ (mg/kg) (95% Conf. Limits) | Relative Potency* (95% Conf. Limits) |
|---|---|---|
| Codeine | 9.2 (6.6–11.9) | |
| Compound A | 48.8 (25.7–72.9) | |
| Codeine + Compound A 1 mg/kg | 3.1 (2.2–4.0) | 2.7 (1.6–6.0) |
| Codeine + Compound A .1 mg/kg | 2.0 (1.0–2.8) | 3.3 (1.8–8.8) |
| Codeine + Compound A .01 mg/kg | 2.6 (1.4–3.8) | 3.0 (1.6–8.9) |
| Codeine + Compound A .001 mg/kg | 3.3 (2.0–5.0) | 2.8 (1.5–7.8) |
| Codeine + Compound A .0001 mg/kg | 5.3 (3.5–8.5) | 1.8 (1.1–4.6) |
| Codeine + Compound A .00001 mg/kg | 4.9 (3.4–6.4) | 1.6 (1.1–2.7) |
| Codeine + Compound A .000001 mg/kg | 5.9 (4.8–7.2) | 1.5 (1.1–2.3) |

*Relative potency of the combination compared to codeine alone, as estimated by parallel line bioassay. Estimates are not exactly the same as those calculated by taking ratios of $ED_{50}$ values because the parallel line estimates are based on *parallel* curves simultaneously fitted to codeine plus any given combination treatment, while the $ED_{50}$ values are based on the best *separately* fitted lines (not precisely parallel).

Inhibition of phenylquinone induced writhing by oxycodone potentiated by 6-chloro-α-methyl carbazole-2-acetic acid was determined by the procedure described hereinabove with one change. For computation of the $ED_{50}$'s and 95% confidence limits by regression analysis and Fieller's Theorem, the analysis was performed on a transformation of the number of writhes made by each mouse. This transformation consisted of the [(square root of the number of writhes) + (square root of the number of writhes + 1)] and is commonly employed with data that are distributed in a Poisson distribution. The results for oxycodone are set forth in Table II.

TABLE II

Summary of Analgesic Effects of Oxycodone and 6-Chloro-α-methylcarbazole-2-acetic acid (Compound A) and Their Combinations in the Phenylquinone Writhing Test

| Compound/Combination | $ED_{50}$ (mg/kg) (95% Conf. Limits) |
|---|---|
| Oxycodone | 0.25 (0.19—0.32) |
| Compound A | 41.9 (33.5—50.0) |
| Oxycodone + Compound A 1 mg/kg | 0.11 (0.08—0.13) |
| Oxycodone + Compound A .1 mg/kg | 0.13 (0.01—0.16) |
| Oxycodone + Compound A.01 mg/kg | 0.13 (0.09—0.18) |

Inhibition of phenylquinone induced writhing by morphine potentiated by 6-chloro-α-methyl-carbazole-2-acetic acid was determined by the procedure described hereinabove with one further change. The statistical method used to estimate the $ED_{50}$'s and 95% confidence limits was that described by J. T. Litchfield and F. Wilcoxon, J. Pharmacol. Exper. Therap. *96*, 99 (1949). This analysis was carried out on the

set of values representing the percent of inhibition of writhing observed at each dose, as defined above, in any given assay. The results for morphine are set forth in Table III.

TABLE III

Summary of Analgesic Effects of Morphine and 6-Chloro-α-methylcarbazole-2-acetic acid (Compound A) and Their Combinations in the Phenylquinone Writhing Test

| Compound/Combination | $ED_{50}$ (mg/kg) (95% Conf. Limits) |
|---|---|
| Morphine | 2.5 (1—4) |
| Compound A | 54 (27—108) |
| Morphine + Compound A 1 mg/kg | 0.96 (0.53—1.73) |

Example 1

Pharmaceutical tablets are prepared from the following formulation by direct compression:

| A. *Ingredients* | mg/tablet | | | |
|---|---|---|---|---|
| 6-chloro-α-methylcarbazole-2-acetic acid | 1 | 5 | 25 | 50 |
| Codeine phosphate | 30 | 30 | 30 | 30 |
| Lactose | 72.5 | 103 | 167.5 | 177 |
| Microcrystalline cellulose | 30 | 40 | 50 | 60 |
| Directly compressible starch | 15 | 20 | 25 | 30 |
| Magnesium Stearate | 1.5 | 2 | 2.5 | 3 |
| weight of tablet: | 150 mg | 200 mg | 300 mg | 350 mg |

| B. *Ingredients* | *mg/tablet* | | | |
|---|---|---|---|---|
| 6-chloro-α-methylcarbazole-2-acetic acid | 1 | 5 | 25 | 50 |
| Codeine phosphate | 15 | 15 | 15 | 15 |
| Lactose | 87.5 | 118 | 182.5 | 192 |
| Microcrystalline cellulose | 30 | 40 | 50 | 60 |
| Directly compressible starch | 15 | 20 | 25 | 30 |
| Magnesium Stearate | 1.5 | 2 | 2.5 | 3 |
| weight of tablet: | 150 mg | 200 mg | 300 mg | 350 mg |

| C. *Ingredients* | *mg/tablet* | | | |
|---|---|---|---|---|
| 6-chloro-α-methylcarbazole-2-acetic acid | 1 | 5 | 25 | 50 |
| Codeine phosphate | 60 | 60 | 60 | 60 |
| Lactose | 72.5 | 103 | 167.5 | 177 |
| Microcrystalline cellulose | 30 | 40 | 50 | 60 |
| Directly compressible starch | 15 | 20 | 25 | 30 |
| Magnesium Stearate | 1.5 | 2 | 2.5 | 3 |
| weight of tablet: | 180 mg | 230 mg | 330 mg | 380 mg |

The active ingredients, the lactose, the cellulose and starch are thoroughly mixed in a suitable container for about 15 minutes. After passing the mixture through a suitable mill, the mixture is mixed again for 10 minutes. The magnesium stearate is added to the mixture and it is mixed for 3 minutes. The mixture is then pressed into tablets.

Example 2

Pharmaceutical tablets are prepared from the following formulation by wet granulation:

| A. *Ingredients* | *mg/tablet* | | | |
|---|---|---|---|---|
| 6-chloro-α-methylcarbazole-2-acetic acid | 1 | 5 | 25 | 30 |
| Codeine phosphate | 15 | 15 | 15 | 15 |
| Pregelatinized Starch | 10 | 15 | 20 | 25 |
| Lactose | 112.5 | 138 | 207.5 | 242 |
| Modified starch | 10 | 25 | 30 | 35 |
| Magnesium Stearate | 1.5 | 2 | 2.5 | 3 |
| weight of tablet: | 150 mg | 200 mg | 300 mg | 350 mg |

| B. *Ingredients* | *mg/tablet* | | | |
|---|---|---|---|---|
| 6-chloro-α-methylcarbazole-2-acetic acid | 1 | 5 | 25 | 30 |
| Codeine phosphate | 30 | 30 | 30 | 30 |
| Pregelatinized Starch | 10 | 15 | 20 | 25 |
| Lactose | 112.5 | 138 | 207.5 | 242 |
| Modified starch | 10 | 25 | 30 | 35 |
| Magnesium Stearate | 1.5 | 2 | 2.5 | 3 |
| weight of tablet: | 165 mg | 215 mg | 315 mg | 365 mg |

| C. *Ingredients* | *mg/tablet* | | | |
|---|---|---|---|---|
| 6-chloro-α-methylcarbazole-2-acetic acid | 1 | 5 | 25 | 30 |
| Codeine phosphate | 60 | 60 | 60 | 60 |
| Pregelatinized Starch | 10 | 15 | 20 | 25 |
| Lactose | 112.5 | 138 | 207.5 | 242 |
| Modified starch | 10 | 25 | 30 | 35 |
| Magnesium Stearate | 1.5 | 2 | 2.5 | 3 |
| weight of tablet: | 195 mg | 245 mg | 345 mg | 395 mg |

The active ingredients, the pregelatinized starch, the lactose and the modified starch are thoroughly mixed and then water is added. The resulting mixture is granulated, dried and passed through a sieve. The resulting granulate is mixed with the magnesium stearate. The mixture is blended in a suitable apparatus until homogeneous and then pressed into tablets.

Example 3

Pharmaceutical capsules are prepared from the following formulation:

| A. *Ingredients* | *mg/capsule* | | | |
|---|---|---|---|---|
| 6-chloro-α-methylcarbazole-2-acetic acid | 1 | 5 | 25 | 50 |
| Codeine phosphate | 30 | 30 | 30 | 30 |
| Lactose DTG | 149 | 200 | 250 | 300 |
| Starch | 20 | 30 | 35 | 40 |
| Talc | 20 | 30 | 35 | 40 |
| capsule fill weight: | 220 mg | 295 mg | 375 mg | 460 mg |

| B. *Ingredients* | *mg/capsule* | | | |
|---|---|---|---|---|
| 6-chloro-α-methylcarbazole-2-acetic acid | 1 | 5 | 25 | 50 |
| Codeine phosphate | 15 | 15 | 15 | 15 |
| Lactose DTG | 149 | 200 | 250 | 300 |
| Starch | 20 | 30 | 35 | 40 |
| Talc | 20 | 30 | 35 | 40 |
| capsule fill weight: | 205 mg | 280 mg | 360 mg | 445 mg |

| C. *Ingredients* | *mg/capsule* | | | |
|---|---|---|---|---|
| 6-chloro-α-methylcarbazole-2-acetic acid | 1 | 5 | 25 | 50 |
| Codeine phosphate | 60 | 60 | 60 | 60 |
| Lactose DTG | 149 | 200 | 250 | 300 |
| Starch | 20 | 30 | 35 | 40 |
| Talc | 20 | 30 | 35 | 40 |
| capsule fill weight: | 250 mg | 325 mg | 405 mg | 490 mg |

Mix all the ingredients for 10 minutes in a suitable mixer. The mixture is milled, remixed and filled into capsules.

Example 4

Pharmaceutical tablets are prepared from the following formulation by direct compression:

| A. *Ingredients* | *mg/tablet* | | | |
|---|---|---|---|---|
| 6-chloro-α-methylcarbazole-2-acetic acid | 1 | 5 | 25 | 50 |
| Morphine sulphate | 30 | 30 | 30 | 30 |
| Lactose DTG | 72.5 | 103 | 167.5 | 177 |
| Microcrystalline cellulose | 30 | 40 | 50 | 60 |
| Directly compressible starch | 15 | 20 | 25 | 30 |
| Magnesium Stearate | 1.5 | 2 | 2.5 | 3 |
| weight of tablet: | 150 mg | 200 mg | 300 mg | 350 mg |

| B. *Ingredients* | *mg/tablet* | | | |
|---|---|---|---|---|
| 6-chloro-α-methylcarbazole-2-acetic acid | 1 | 5 | 25 | 50 |
| Morphine sulphate | 15 | 15 | 15 | 15 |
| Lactose DTG | 87.5 | 118 | 182.5 | 192 |
| Microcrystalline cellulose | 30 | 40 | 50 | 60 |
| Directly compressible starch | 15 | 20 | 25 | 30 |
| Magnesium Stearate | 1.5 | 2 | 2.5 | 3 |
| weight of tablet: | 150 mg | 200 mg | 300 mg | 350 mg |

| C. *Ingredients* | *mg/tablet* | | | |
|---|---|---|---|---|
| 6-chloro-α-methylcarbazole-2-acetic acid | 1 | 5 | 25 | 50 |
| Morphine sulphate | 60 | 60 | 60 | 60 |
| Lactose DTG | 72.5 | 103 | 167.5 | 177 |
| Microcrystalline cellulose | 30 | 40 | 50 | 60 |
| Directly compressible starch | 15 | 20 | 25 | 30 |
| Magnesium Stearate | 1.5 | 2 | 2.5 | 3 |
| weight of tablet: | 180 mg | 230 mg | 330 mg | 380 mg |

The active ingredients, the lactose, the cellulose and starch are thoroughly mixed in a suitable container for about 15 minutes. After passing the mixture through a suitable mill, the mixture is mixed again for 10 minutes. The magnesium stearate is added to the mixture and it is mixed for 3 minutes. The mixture is then pressed into tablets.

Example 5

Pharmaceutical tablets are prepared from the following formulation by wet granulation:

| A. *Ingredients* | *mg/tablet* | | | |
|---|---|---|---|---|
| 6-chloro-α-methylcarbazole-2-acetic acid | 1 | 5 | 25 | 30 |
| Morphine sulphate | 15 | 15 | 15 | 15 |
| Pregelatinized Starch | 10 | 15 | 20 | 25 |
| Lactose | 112.5 | 138 | 207.5 | 242 |
| Modified starch | 10 | 25 | 30 | 35 |
| Magnesium Stearate | 1.5 | 2 | 2.5 | 3 |
| weight of tablet: | 150 mg | 200 mg | 300 mg | 350 mg |

| B. *Ingredients* | *mg/tablet* | | | |
|---|---|---|---|---|
| 6-chloro-α-methylcarbazole-2-acetic acid | 1 | 5 | 25 | 30 |
| Morphine sulphate | 30 | 30 | 30 | 30 |
| Pregelatinized Starch | 10 | 15 | 20 | 25 |
| Lactose | 112.5 | 138 | 207.5 | 242 |
| Modified starch | 10 | 25 | 30 | 35 |
| Magnesium Stearate | 1.5 | 2 | 2.5 | 3 |
| weight of tablet: | 165 mg | 215 mg | 315 mg | 365 mg |

| C. *Ingredients* | *mg/tablet* | | | |
|---|---|---|---|---|
| 6-chloro-α-methylcarb-azole-2-acetic acid | 1 | 5 | 25 | 30 |
| Morphine sulphate | 60 | 60 | 60 | 60 |
| Pregelatinized Starch | 10 | 15 | 20 | 25 |
| Lactose | 112.5 | 138 | 207.5 | 242 |
| Modified starch | 10 | 25 | 30 | 35 |
| Magnesium Stearate | 1.5 | 2 | 2.5 | 3 |
| weight of tablet: | 195 mg | 245 mg | 345 mg | 395 mg |

The active ingredients, the pregelatinized starch, the lactose and the modified starch are thoroughly mixed and then water is added. The resulting mixture is granulated, dried and passed through a sieve. The resulting granulate is mixed with the magnesium stearate. The mixture is blended on a suitable apparatus until homogeneous and pressed into tablets.

Example 6

Pharmaceutical capsules are prepared from the following formulation:

| A. *Ingredients* | *mg/capsule* | | | |
|---|---|---|---|---|
| 6-chloro-α-methylcarb-azole-2-acetic acid | 1 | 5 | 25 | 50 |
| Morphine sulfate | 30 | 30 | 30 | 30 |
| Lactose DTG | 149 | 200 | 250 | 300 |
| Starch | 20 | 30 | 35 | 40 |
| Talc | 20 | 30 | 35 | 40 |
| capsule fill weight: | 220 mg | 295 mg | 375 mg | 460 mg |

| B. *Ingredients* | *mg/capsule* | | | |
|---|---|---|---|---|
| 6-chloro-α-methylcarb-azole-2-acetic acid | 1 | 5 | 25 | 50 |
| Morphine sulfate | 15 | 15 | 15 | 15 |
| Lactose DTG | 149 | 200 | 250 | 300 |
| Starch | 20 | 30 | 35 | 40 |
| Talc | 20 | 30 | 35 | 40 |
| capsule fill weight: | 205 mg | 280 mg | 360 mg | 445 mg |

| C. *Ingredients* | *mg/capsule* | | | |
|---|---|---|---|---|
| 6-chloro-α-methylcarbazole-2-acetic acid | 1 | 5 | 25 | 50 |
| Morphine sulfate | 60 | 60 | 60 | 60 |
| Lactose DTG | 149 | 200 | 250 | 300 |
| Starch | 20 | 30 | 35 | 40 |
| Talc | 20 | 30 | 35 | 40 |
| capsule fill weight: | 250 mg | 325 mg | 405 mg | 490 mg |

Mix all the ingredients for 10 minutes in a suitable mixer. The mixture is milled, remixed and filled into capsules.

Example 7

Pharmaceutical tablets are prepared from the following formulation by direct compression:

| A. *Ingredients* | *mg/tablet* | | | |
|---|---|---|---|---|
| 6-chloro-α-methylcarbazole-2-acetic acid | 1 | 5 | 25 | 50 |
| Oxycodone | 1 | 1 | 1 | 1 |
| Lactose | 132 | 148 | 162.5 | 167 |
| Microcrystalline cellulose | 30 | 30 | 40 | 50 |
| Directly compressible starch | 15 | 15 | 20 | 30 |
| Magnesium Stearate | 1 | 1 | 1.5 | 2 |
| weight of tablet: | 180 mg | 200 mg | 250 mg | 300 mg |

| B. *Ingredients* | *mg/tablet* | | | |
|---|---|---|---|---|
| 6-chloro-α-methylcarbazole-2-acetic acid | 1 | 5 | 25 | 50 |
| Oxycodone | 5 | 5 | 5 | 5 |
| Lactose | 133 | 149 | 163.5 | 168 |
| Microcrystalline cellulose | 30 | 30 | 40 | 50 |
| Directly compressible starch | 15 | 15 | 20 | 30 |
| Magnesium Stearate | 1 | 1 | 1.5 | 2 |
| weight of tablet: | 185 mg | 205 mg | 255 mg | 305 mg |

The active ingredients, the lactose, the cellulose and starch are thoroughly mixed in a suitable container for about 15 minutes. After passing the mixture through a suitable mill, the mixture is mixed again for 10 minutes. The magnesium stearate is added to the mixture and it is mixed for 3 minutes. The mixture is then pressed into tablets.

Example 8

Pharmaceutical tablets are prepared from the following formulation by wet granulation:

| A. *Ingredients* | *mg/tablet* | | | |
|---|---|---|---|---|
| 6-chloro-α-methylcarbazole-2-acetic acid | 1 | 5 | 25 | 50 |
| Oxycodone | 1 | 1 | 1 | 1 |
| Pregelatinized Starch | 5 | 10 | 15 | 20 |
| Lactose | 162 | 167.5 | 187 | 201.5 |
| Modified Starch | 105 | 15 | 20 | 25 |
| Magnesium Stearate | 1 | 1.5 | 2.0 | 2.5 |
| weight of tablet: | 180 mg | 200 mg | 250 mg | 300 mg |

| B. *Ingredients* | *mg/tablet* | | | |
|---|---|---|---|---|
| 6-chloro-α-methylcarbazole-2-acetic acid | 1 | 5 | 25 | 50 |
| Oxycodone | 5 | 5 | 5 | 5 |
| Lactose | 162 | 167.5 | 187 | 201.5 |
| Modified Starch | 10 | 15 | 20 | 25 |
| Pregelatinized Starch | 5 | 10 | 15 | 20 |
| Magnesium Stearate | 1 | 1.5 | 2 | 2.5 |
| weight of tablet: | 184 mg | 204 mg | 254 mg | 304 mg |

The active ingredients, the pregelatinized starch, the lactose and the modified starch are thoroughly mixed and then water is added. The resulting mixture is granulated, dried and passed through a sieve. The resulting granulate is mixed with the magnesium stearate. The mixture is blended on a suitable apparatus until homogeneous and pressed into tablets.

Example 9

Pharmaceutical capsules are prepared from the following formulation:

| A. *Ingredients* | *mg/capsule* | | | |
|---|---|---|---|---|
| 6-chloro-α-methylcarbazole-2-acetic acid | 1 | 5 | 25 | 50 |
| Oxycodone | 1 | 1 | 1 | 1 |
| Lactose | 158 | 184 | 204 | 219 |
| Starch | 20 | 30 | 35 | 40 |
| Talc | 20 | 30 | 35 | 40 |
| capsule fill weight: | 200 mg | 250 mg | 300 mg | 350 mg |

| B. *Ingredients* | *mg/capsule* | | | |
|---|---|---|---|---|
| 6-chloro-α-methylcarbazole-2-acetic acid | 1 | 5 | 25 | 50 |
| Oxycodone | 5 | 5 | 5 | 5 |
| Lactose | 158 | 184 | 204 | 219 |
| Starch | 20 | 30 | 35 | 40 |
| Talc | 20 | 30 | 35 | 40 |
| capsule fill weight: | 204 mg | 254 mg | 304 mg | 354 mg |

Mix all the ingredients for 10 minutes in a suitable mixer. The mixture is milled, remixed and filled into capsules.

**Claims**

1. An analgesic composition containing as active ingredients an opiate alkaloid compound of formula

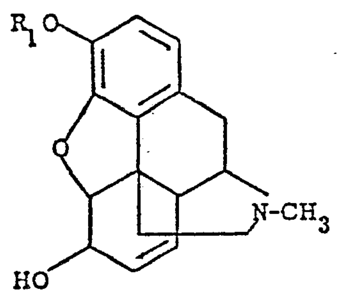

I, or

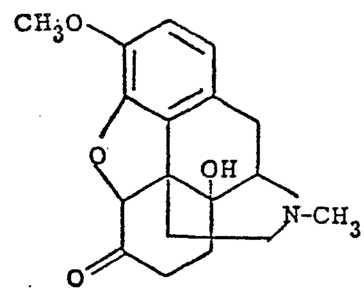

II

wherein $R_1$ is hydrogen or alkyl containing 1—7 carbon atoms, or a pharmaceutically acceptable acid addition salt thereof; and the carbazole compound 6-chloro-α-methyl-carbazole-2-acetic acid of the formula

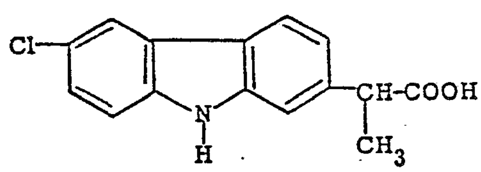

III

or a salt thereof with a pharmaceutically acceptable base.

2. A composition in accordance with claim 1 containing 0,05 to 90 percent by weight of the active ingredients.

3. A composition in accordance with claim 1 or claim 2, wherein the weight ratio of 6-chloro-α-methyl-carbazole-2-acetic acid and said opiate alkaloid compound is from 1:10 to 10:1.

4. A composition in accordance with claim 3, wherein the weight ratio of 6-chloro-α-methyl-carbazole-2-acetic acid and said opiate alkaloid compound is from 1:5 to 5:1.

5. A composition in accordance with any one of claims 1 to 4, wherein the opiate alkaloid compound is morphine or oxycodone.

6. A composition in accordance with any one of claims 1 to 4, wherein the opiate alkaloid compound is codeine.

7. A composition in accordance with claim 6, wherein the weight ratio of 6-chloro-α-methyl-carbazole-2-acetic acid and codeine is 5:2.

**Patentansprüche**

1. Ein analgetisches Präparat enthaltend als Wirkstoffe eine Opiatalkaloidverbindung der Formel

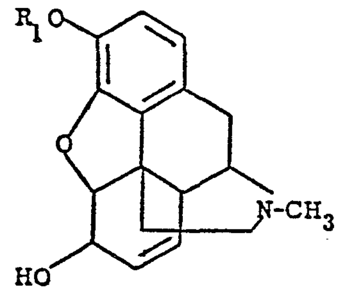 I oder 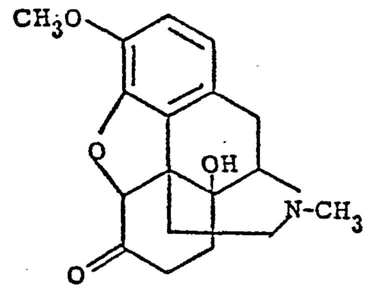 II

worin $R_1$ Wasserstoff oder Alkyl mit 1 bis 7 Kohlenstoffatomen ist, oder ein pharmazeutisch akzeptables Säureadditionssalz davon, und eine Carbazolverbindung 6-Chlor-α-methyl-carbazol-2-essigsäure der Formel

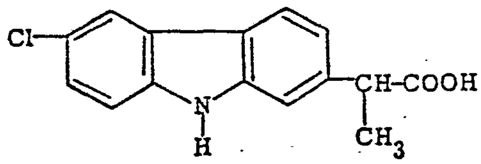 III

oder ein Salz davon mit einer pharmazeutisch akzeptablen Base.

2. Ein Präparat gemäss Anspruch 1, enthaltend von 0,05 bis 90 Gewichtprozente der Wirkstoffe.

3. Ein Präparat gemäss Anspruch 1 oder Anspruch 2, worin das Gewichtsverhältnis der 6-Chlor-α-methyl-carbazol-2-essigsäure und der besagten Opiatalkaloidverbindung zwischen 1:10 und 10:1 liegt.

4. Ein Präparat gemäss Anspruch 3, worin das Gewichtsverhältnis der 6-Chlor-α-methyl-carbazol-2-essigsäure und der besagten Opiatalkaloidverbindung zwischen 1:5 und 5:1 liegt.

5. Ein Präparat gemäss einem der Ansprüche 1 bis 4, worin die Opiatalkaloidverbindung Morphin oder Oxycodon ist.

6. Ein Präparat gemäss einem der Ansprüche 1 bis 4, worin die Opiatalkaloidverbindung Codein ist.

7. Ein Präparat gemäss Anspruch 6, worin das Gewichtsverhältnis zwischen der 6-Chlor-α-methyl-carbazol-2-essigsäure und Codein 5:2 ist.

**Revendications**

1. Une composition analgésique contenant comme ingrédients actifs un composé alcaloïde opiacé de formule:

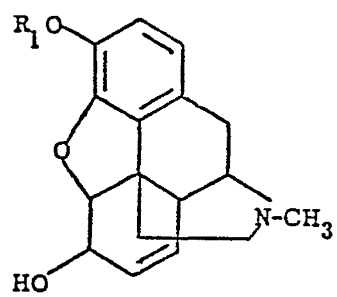 I, ou 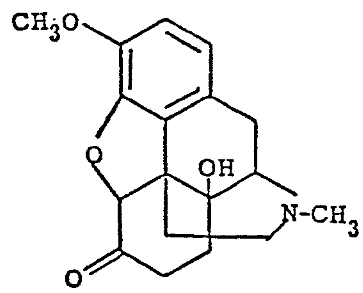 II

où $R_1$ est un hydrogène ou un alkyle contenant 1 à 7 atomes de carbone, ou un sel d'addition d'acide convenant en pharmacie correspondant; et le composé de carbazole qu'est l'acide 6-chloro-α-méthyl-carbazole-2-acétique de formule:

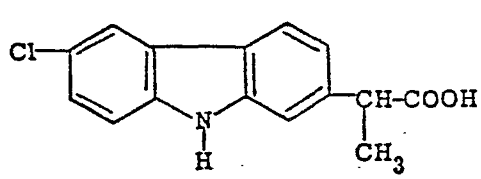 III

ou un de ses sels formé avec une base acceptable en pharmacie.

2. Une composition selon la revendication 1, contenant 0,05 à 90% en poids des ingrédients actifs.

3. Une composition selon la revendication 1 ou la revendication 2 dans laquelle le rapport pondéral de l'acide 6-chloro-α-méthyl-carbazole-2-acétique et dudit composé alcaloïde opiacé est de 1/10 à 10/1.

4. Une composition selon la revendication 3 dans laquelle le rapport pondéral de l'acide 6-chloro-α-méthyl-carbazole-2-acétique et dudit composé alcaloïde opiacé est de 1/5 à 5/1.

5. Une composition selon l'une quelconque des revendications 1 à 4 dans laquelle le composé alcaloïde opiacé est la morphine ou l'oxycodone.

6. Une composition selon l'une quelconque des revendications 1 à 4 dans laquelle le composé alcaloïde opiacé est la codéine.

7. Une composition selon la revendication 6 dans laquelle le rapport pondéral de l'acide 6-chloro-α-méthyl-carbazole-2-acétique et de la codéine est de 5/2.